(19) Europäisches Patentamt
European Patent Office
Office européen des brevets

(11) EP 2 149 386 A1

(12) DEMANDE DE BREVET EUROPEEN

(43) Date de publication:
03.02.2010 Bulletin 2010/05

(51) Int Cl.:
*A61M 25/09* (2006.01)

(21) Numéro de dépôt: 08161434.9

(22) Date de dépôt: 30.07.2008

(84) Etats contractants désignés:
AT BE BG CH CY CZ DE DK EE ES FI FR GB GR
HR HU IE IS IT LI LT LU LV MC MT NL NO PL PT
RO SE SI SK TR
Etats d'extension désignés:
AL BA MK RS

(71) Demandeur: UNIVERSITE LIBRE DE BRUXELLES
1050 Brussels (BE)

(72) Inventeurs:
• Deviere, Jacques
1470 Genappe (BE)
• Cauche, Nicolas
1160 Bruxelles (BE)
• Delchambre, Alain
1170 Bruxelles (BE)
• Dugardeyn, Sonia
7090 Braine le Comte (BE)

(74) Mandataire: pronovem
Office Van Malderen
Avenue Josse Goffin 158
1082 Bruxelles (BE)

(54) **Guide pour cathétérisme**

(57) La présente invention concerne l'utilisation d'un guide pour le cathétérisme sélectif de structures anatomiques et le placement d'instruments thérapeutiques.

Fig. 1

EP 2 149 386 A1

## Description

### Objet de l'invention

La présente invention concerne l'utilisation d'un guide pour le cathétérisme sélectif de structures anatomiques et le placement d'instruments thérapeutiques.

### Problème technique

Le passage d'un guide souple appelé "guide wire" dans les différents conduits naturels du corps humain tels que ceux présents dans les systèmes digestif, hépato-pancréatique, urinaire, génital, respiratoire, cardiovasculaire est généralement nécessaire pour introduire des instruments thérapeutiques (« medical devices ») jusqu'au site à traiter. Le guide doit donc traverser les différents conduits jusqu'au site à traiter. Dans tous ces conduits, des obstacles peuvent être présents empêchant la bonne navigation du guide et ne permettant pas d'accéder facilement au site à traiter. Les obstacles peuvent être de différentes natures, naturels ou non, comme constitués par des corps étrangers, des calculs, des plis de la muqueuse, des tumeurs ou même du fait de l'anatomie sinueuse du conduit. Afin de contourner ces obstacles et de naviguer dans ces conduits sinueux, les guides actuels sont pourvus d'une extrémité flexible. En effet, lorsque le guide est confronté à un de ces obstacles, il peut être dévié et venir s'impacter ou toucher les parois du conduit ce qui peut conduire à une blessure (oedème) et à une détérioration du guide rendant la suite de la navigation plus difficile. Des solutions ont été proposées dans les différents documents de l'état de la technique tels que le "Loop Tip Wire Guide" (décrit dans le document WO 2006/039217). Cependant, les solutions présentes actuellement ne permettent pas de résoudre totalement ce problème technique.

### Principaux éléments caractéristiques

La présente invention permet d'éviter les blessures ou contusions dues à l'extrémité acérée du guide et donc aux muqueuses et aux parois endothéliales, et permet de faciliter la navigation du guide grâce à la forme particulière de son extrémité.

Selon l'invention, le guide pour cathétérisme est muni à son extrémité distale d'une excroissance ou embout présentant une forme tridimensionnelle, de préférence axisymétrique, autour de l'axe de révolution dudit guide. Cette forme tridimensionnelle peut être par exemple ovoïdale, sphéroïdale, ellipsoïde. De préférence cette forme ne présente pas d'arrête ou d'angle aigu ou présente un profil perpendiculaire à l'axe longitudinal qui possède un rayon de courbure quasi constant. Par quasi constant on entend une variation de 50 à 200%.

Plus particulièrement, le guide est **caractérisé en ce que** l'excroissance ou embout présente dans le plan perpendiculaire à l'axe longitudinal dudit guide au moins une dimension supérieure au diamètre dudit guide. De préférence, au moins une dimension et de préférence une dimension dans un plan perpendiculaire à l'axe longitudinal et en particulier la largeur de l'excroissance est significativement supérieure au diamètre du corps du guide.

Par significativement, on entend une augmentation d'au moins 25%, de préférence 50%, de préférence 100%.

Selon une autre alternative, l'excroissance présente des dimensions significativement plus importantes que le corps du guide tant dans le sens radial qu'axial.

L'excroissance ou l'embout du guide peut être solidarisé de manière permanente au corps dudit guide.

Alternativement, l'excroissance ou l'embout du guide peut être détachable du corps dudit guide. La désolidarisation pourra avantageusement se produire au sein même du corps.

L'excroissance peut être détachable ou non du guide au sein même du corps. Cette excroissance pourra bien entendu également être détachable à l'extérieur du corps humain. A titre d'exemple la libération de l'excroissance du guide pourra se faire par l'action d'un dispositif médical déjà présent sur le guide.

Le matériau de l'excroissance ou de l'embout du guide est avantageusement déformable ou extensible par déploiement.

Alternativement, le matériau de l'excroissance ou de l'embout du guide peut ne pas être déformable.

Plus particulièrement, cette excroissance peut être réalisée en matériau métallique, organique (par exemple polymère, élastomère), céramique ou composite. L'excroissance devra être de préférence biocompatible (comme par exemple décrit dans la norme EN ISO 10993-1) et inaltérable par le milieu environnant.

Cette excroissance peut-être pleine, creuse ou traversée par un ou plusieurs orifices.

Sa surface extérieure sera de préférence traitée pour obtenir un coefficient de frottement plus faible que l'environnement anatomique et donc présenter une rugosité améliorée de manière à permettre et à faciliter la navigation.

L'excroissance peut être réalisée dans les mêmes matériaux que ceux utilisés pour la réalisation du guide ou faire partie directement du guide.

La présente invention permet avantageusement une utilisation unique du guide pour cathéter.

Selon une autre forme d'exécution on peut envisager également que l'excroissance ne soit pas présente sur le guide avant même qu'il soit introduit dans le corps humain, elle n'apparaît que dans le corps humain, par exemple par l'action d'une membrane gonflable.

### Description de plusieurs formes d'exécution préférées de l'invention

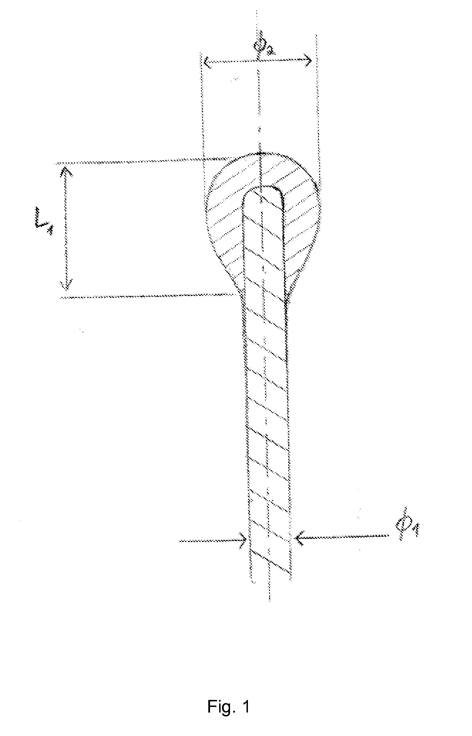

Les figures 1, 2, 3 et 4 représentent chacune une forme préférentielle de l'excroissance présente à l'extrémité du guide. Ces excroissances possèdent une forme tridimensionnelle et de préférence axisymétrique autour de l'axe de révolution du corps du guide. Au moins une dimension et de préférence une dimension dans un plan perpendiculaire à l'axe longitudinal et en particulier la largeur de l'excroissance est significativement supérieure au diamètre du corps du guide.

Par significativement, on entend une augmentation d'au moins 25%, de préférence d'au moins 50% et de préférence de 100%.

Dans une première forme d'exécution représentée à la figure 1, l'excroissance dépasse significativement le corps du guide tant dans le sens axial que radial.

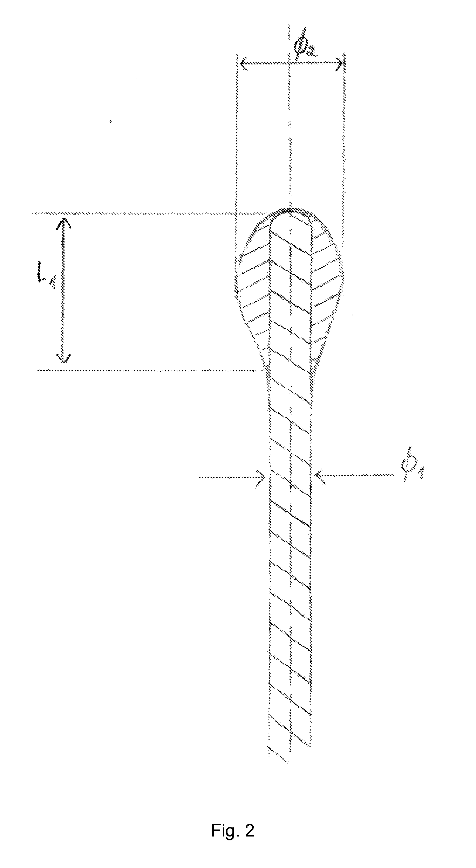

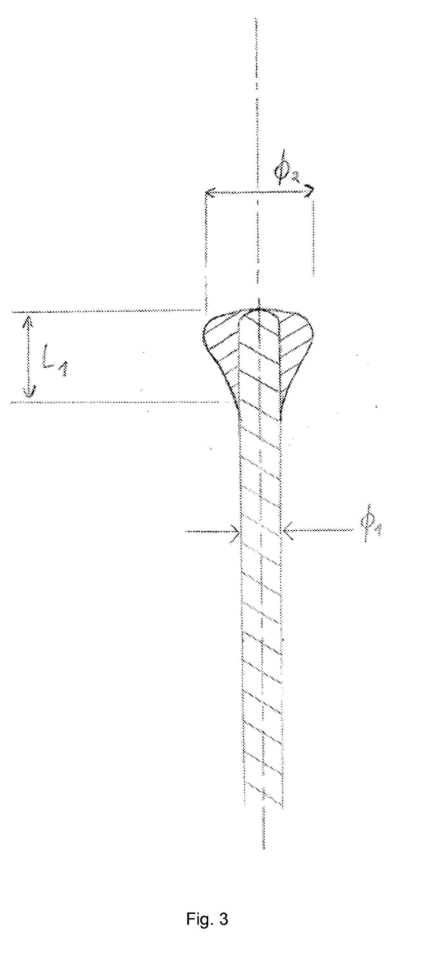

Dans une autre forme d'exécution, plus particulièrement représentée aux figures 2 et 3, la largeur de l'excroissance ne dépasse significativement le corps du guide que dans le sens radial.

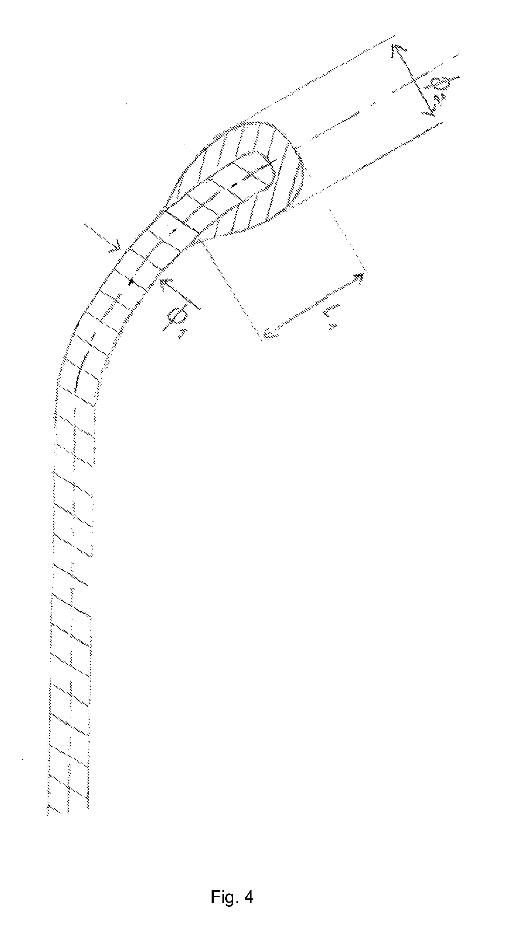

Dans une autre forme d'exécution, plus particulièrement représentée à la figure 4, le guide est courbé, l'axe de révolution de l'excroissance étant de préférence l'axe du guide lorsque celui-ci est redressé (c'est-à-dire en position non courbée).

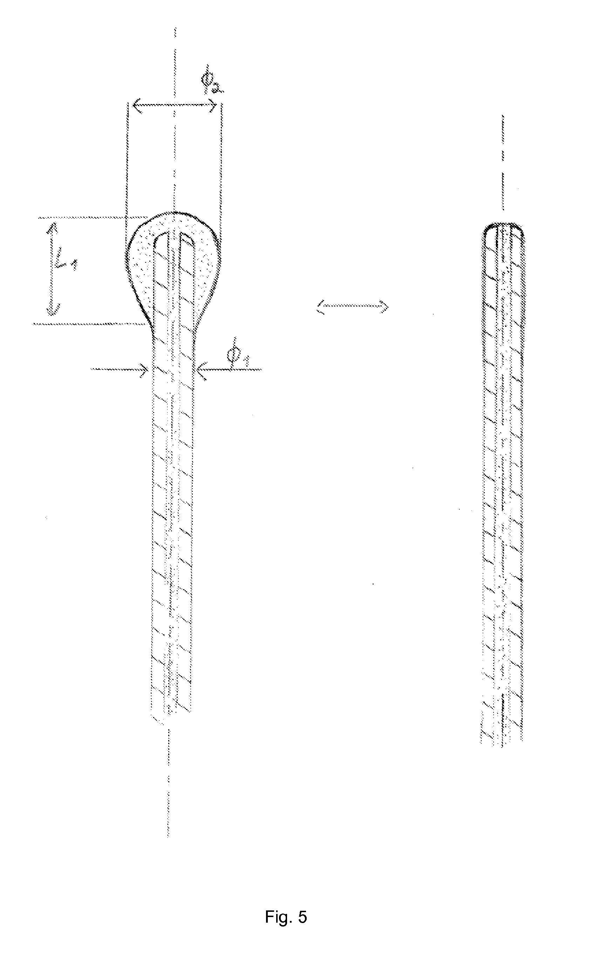

Dans une autre forme d'exécution telle que représentée à la figure 5, l'excroissance est déformable ou extensible permettant une modification de forme et/ou de volume, en permettant une transition d'un état déployé vers un état non déployé ou inversement, l'état déployé étant défini par le fait qu'au moins une dimension et en particulier une dimension perpendiculaire à l'axe longitudinal et de préférence la largeur de l'excroissance est significativement supérieure au diamètre du corps du guide tandis que la position non déployée correspond à un état où l'excroissance ne présente pas de dimensions supérieures dans le plan perpendiculaire à l'axe longitudinal, au diamètre du corps du guide.

Dans une dernière forme d'exécution préférée de l'invention, l'excroissance est préférentiellement constituée par un élément extensible ou gonflable tel qu'une membrane ou un ballon qui peut être gonflé par un gaz ou un fluide. Dans ce cas, l'excroissance est obtenue par exemple par simple injection sous pression d'un fluide au travers d'un canal interne au guide. D'autres modes d'exécution permettent d'obtenir ce même résultat.

La position non déployée permet d'accéder à des conduits naturels ou opératoires où l'excroissance empêcherait le passage du guide muni d'une excroissance déployée et ce, du fait de ses dimensions. De plus, dans cette forme préférée d'exécution, la largeur maximale de l'excroissance peut être préférentiellement adaptée en fonction de l'anatomie du patient et ainsi permettre une navigation optimale. De plus, dans cette forme préférée d'exécution, l'excroissance peut être préférentiellement utilisée pour dégager les conduits.

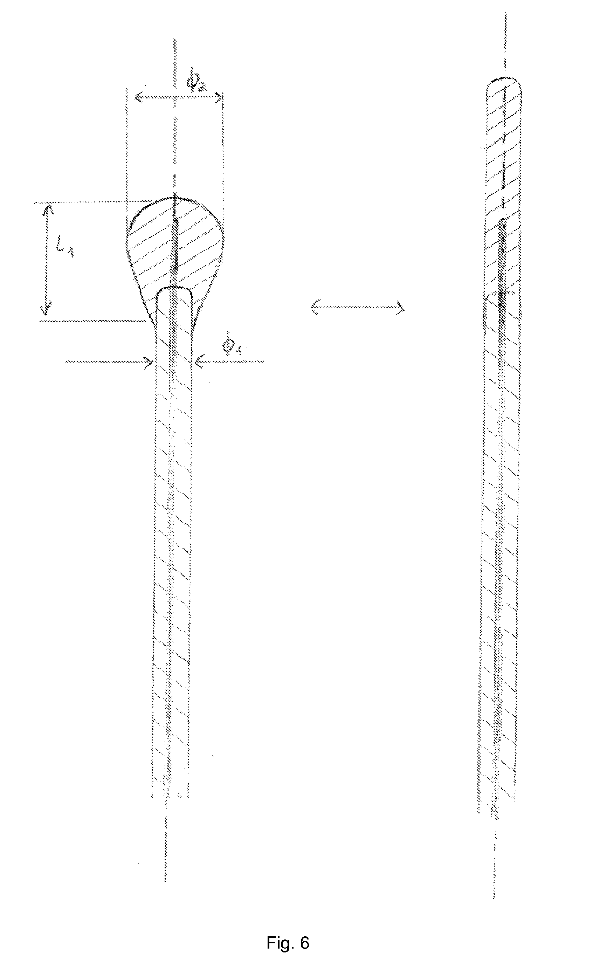

Selon une forme d'exécution représentée à la figure 6, l'excroissance est extensible ou déformable. Par extensible, on entend une modification du volume de l'excroissance tandis que par déformable, on entend une modification de sa forme.

Avantageusement, l'excroissance peut passer d'un état déployé à un état non déployé par modification de sa forme et/ou de son volume. L'état déployé étant défini par le fait qu'au moins une dimension et en particulier une dimension perpendiculaire à l'axe longitudinal et de préférence la largeur de l'excroissance est significativement supérieure au diamètre du corps du guide tandis que la position non déployée correspond à un état où l'excroissance ne présente pas de dimensions supérieures au diamètre du corps du guide.

Selon une autre forme d'exécution préférée, l'excroissance peut être réalisée en un matériau à mémoire de forme telle que les « Shape Memory Polymers » qui modifieront leur état ou leur forme par élévation de la température du milieu ambiant. Cette élévation de température peut se produire tout simplement par l'application d'un moyen externe tel qu'un courant électrique ou simplement par l'influence de la température corporelle.

**Exemples** :

1. Cathétérisme sélectif de la voie biliaire.

Le cathétérisme sélectif de la voie biliaire au travers de la papille de Vater est techniquement difficile et peut se faire soit directement avec un cathéter soit à l'aide d'un guide. Le sphincter d'Oddi est recouvert d'une muqueuse et, lorsque le cathétérisme n'est pas réussi lors des premières manoeuvres, la manipulation répétée de cathéters ou de guide qui s'impactent dans la muqueuse, créent un oedème, rendant la procédure plus difficile.

Le type de guide décrit plus haut, muni d'une extrémité atraumatique et arrondie, ne disposant d'aucune angulation, permet de réduire le traumatisme de la muqueuse et l'oedème qui en résulte. De même, il aide l'extrémité flexible du guide à s'adapter à la forme en S de la papille, afin de trouver son chemin vers la voie biliaire.

Des guides modifiés selon la présente invention ont été testés dans des modèles animaux. Ces tests ont révélé que cette forme particulière de réalisation de la présente invention permet de réduire significativement la tendance à l'impaction de l'extrémité distale du guide dans des structures complexes.

Afin de pouvoir continuer à utiliser le guide, dans des conduits plus étroits, l'excroissance est détachée volontairement du guide, à l'intérieur des animaux, par l'action du cathéter sur le guide.

L'excroissance utilisée pour ce cathétérisme est une forme similaire à celle représentée à la figure 2. Cette excroissance est détachable du corps du guide par l'action du "medical device". Le matériau testé constituant l'excroissance est le caoutchouc naturel. Les dimensions de l'excroissance pour ce test sont :

$\Phi_1$ : Diamètre du guide, à son extrémité flexible.
$\Phi_1$ = 0.035" (0.889 mm) dans cet exemple.

$\Phi_2$ : Largeur maximale de l'excroissance.
$\Phi_2$ = 1.7 mm dans cet exemple.

$L_1$ : Hauteur de l'excroissance.
$L_1$ = 2.5 mm dans cet exemple.

2. Cathétérisme sélectif du pancréas.

Le cathétérisme sélectif du pancréas est techniquement difficile et peut se faire soit directement avec un cathéter, soit à l'aide d'un guide. Le sphincter d'Oddi est recouvert d'une muqueuse et, lorsque le cathétérisme n'est pas réussi lors des premières manoeuvres, la manipulation répétée de cathéters ou de guide qui s'impactent dans la muqueuse, créent un oedème, rendant la procédure plus difficile. Le type de guide décrit plus haut, muni d'une extrémité atraumatique et arrondie, ne disposant d'aucune angulation, permet de réduire le traumatisme à la muqueuse et l'oedème qui en résulte. De même, il aide l'extrémité flexible du guide à s'adapter à la forme en S de la papille, afin de trouver son chemin vers le pancréas.

Des guides modifiés selon la présente invention ont été testés dans des modèles animaux. Ces tests ont révélé que cette forme particulière de réalisation de la présente invention permet de réduire significativement la tendance à l'impaction de l'extrémité distale du guide dans des structures complexes.

L'excroissance utilisée pour ce cathétérisme est une forme sphérique. Cette excroissance n'est pas détachable du corps du guide. Le matériau testé constituant l'excroissance est l'acier inoxydable 316L. Les dimensions de l'excroissance pour ce test sont :

$\Phi_1$ : Diamètre du guide, à son extrémité flexible.
$\Phi_1$ = 0.035" (0.889 mm) dans cet exemple.

$\Phi_2$ : Largeur maximale de l'excroissance.
$\Phi_2$ = 1.5 mm dans cet exemple.

$L_1$ : Hauteur de l'excroissance.
$L_1$ = 1 mm dans cet exemple.

Dimensions de l'excroissance dans le cas du cathétérisme de la voie biliaire et du pancréas :

$$1.2\Phi_1 \quad < \quad \Phi_2 \quad < \quad 3 \text{ mm}$$

$$0.5 \text{ mm} \quad < \quad L_1 \quad < \quad 5 \text{ mm}$$

$\Phi_1$ : Diamètre du guide, à son extrémité flexible.
$\Phi_2$ : Largeur maximale de l'excroissance.
$L_1$ : Hauteur de l'excroissance

**Revendications**

1. Guide pour cathétérisme muni à son extrémité distale d'une excroissance ou embout présentant une forme tridimensionnelle, de préférence axisymétrique, autour de l'axe de révolution dudit guide.

2. Guide selon la revendication 1, **caractérisé en ce que** l'excroissance ou embout présente au moins une dimension et en particulier une dimension dans un plan perpendiculaire à l'axe longitudinal et de préférence la largeur de l'excroissance qui est significativement supérieure au diamètre du corps du guide.

3. Guide selon la revendication 2, **caractérisé en ce qu'**une dimension et en particulier une dimension dans le plan perpendiculaire à l'axe longitudinal et de préférence la largeur de l'excroissance est supérieure d'au moins 25%, de préférence de 50%, de préférence de 100% au diamètre du corps du guide.

4. Guide selon l'une quelconque des revendications précédentes, **caractérisé en ce que** ladite excroissance ou ledit embout est solidarisé au corps dudit guide.

5. Guide selon l'une quelconque des revendications précédentes, **caractérisé en ce que** ladite excroissance ou ledit embout est détachable du corps dudit guide.

6. Guide selon l'une quelconque des revendications précédentes, **caractérisé en ce que** l'excroissance, ladite excroissance ou ledit embout est déformable ou extensible.

7. Guide selon la revendication 6, **caractérisé en ce que** ladite excroissance ou ledit embout permet la transition entre un état déployé et un état non déployé par modification de sa forme et/ou de son volume.

Fig. 1

Fig. 2

Fig. 3

Fig. 4

Fig. 5

Fig. 6

**Europäisches Patentamt**
**European Patent Office**
**Office européen des brevets**

# RAPPORT DE RECHERCHE EUROPEENNE

Numéro de la demande

EP 08 16 1434

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | WO 94/13350 A (SCHNEIDER USA INC [US]) 23 juin 1994 (1994-06-23) * page 1, ligne 7 - page 5, ligne 3 * * figures 1,2,4-10 * | 1-4 | INV. A61M25/09 |
| X | EP 0 778 042 A (TARGET THERAPEUTICS INC [US]) 11 juin 1997 (1997-06-11) * abrégé * * colonne 8, ligne 6 - colonne 9, ligne 28 * * figures 1,3 * | 1-4 | |
| X | US 4 991 602 A (AMPLATZ CURTIS A [US] ET AL) 12 février 1991 (1991-02-12) * colonne 2, ligne 17 - colonne 3, ligne 68 * * figures 1-3 * | 1-4 | |
| X | EP 0 515 201 A (BARD INC C R [US]) 25 novembre 1992 (1992-11-25) * abrégé * * figures 1,3 * * colonne 3, ligne 45 - colonne 9, ligne 24 * | 1-4 | DOMAINES TECHNIQUES RECHERCHES (IPC) A61M |
| X | EP 0 410 557 A (INTERVENTIONAL TECHNOLOGIES [US]) 30 janvier 1991 (1991-01-30) * abrégé * * figures 1-3 * * colonne 5, ligne 1-26 * * colonne 7, ligne 2-14 * | 1-4 | |
| X | WO 2004/093659 A (ATRICURE INC [US]) 4 novembre 2004 (2004-11-04) * figures 2,7a,7b,7c,7d * * alinéa [0066] * | 1-4 | |

-/--

Le présent rapport a été établi pour toutes les revendications

| Lieu de la recherche | Date d'achèvement de la recherche | Examinateur |
|---|---|---|
| Munich | 13 novembre 2008 | Rodrigues, Elodie |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Europäisches
Patentamt

European
Patent Office

Office européen
des brevets

## RAPPORT DE RECHERCHE EUROPEENNE

Numéro de la demande

EP 08 16 1434

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | EP 1 803 484 A (CORDIS CORP [US]) 4 juillet 2007 (2007-07-04) * figures 4,5 * ----- | 1-4 | |
| X | US 2003/163064 A1 (VRBA ANTHONY C [US] ET AL) 28 août 2003 (2003-08-28) * figures 5-9 * * alinéas [0004] - [0006] * * alinéas [0011] - [0015] * * alinéas [0033] - [0036] * ----- | 1-4,6,7 | |
| X | US 2008/051721 A1 (CARTER MATTHEW P [US] ET AL) 28 février 2008 (2008-02-28) * abrégé * * figures 1-3c * * alinéas [0001] - [0044] * ----- | 1-3,5 | |
| A | EP 1 092 449 A (USAMINANOTECHNOLOGY INC [JP]) 18 avril 2001 (2001-04-18) * figures 14a,14b,14c,15,16 * ----- | 7 | |
| X | US 2005/273147 A1 (ISRAEL HENRY M [IL]) 8 décembre 2005 (2005-12-08) * figures 2a,2b * * alinéas [0026] - [0030] * ----- | 1-4,6,7 | DOMAINES TECHNIQUES RECHERCHES (IPC) |

Le présent rapport a été établi pour toutes les revendications

| Lieu de la recherche | Date d'achèvement de la recherche | Examinateur |
|---|---|---|
| Munich | 13 novembre 2008 | Rodrigues, Elodie |

CATEGORIE DES DOCUMENTS CITES

X : particulièrement pertinent à lui seul
Y : particulièrement pertinent en combinaison avec un
autre document de la même catégorie
A : arrière-plan technologique
O : divulgation non-écrite
P : document intercalaire

T : théorie ou principe à la base de l'invention
E : document de brevet antérieur, mais publié à la
date de dépôt ou après cette date
D : cité dans la demande
L : cité pour d'autres raisons

................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**    EP 08 16 1434

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

13-11-2008

| Document brevet cité au rapport de recherche | | Date de publication | Membre(s) de la famille de brevet(s) | | Date de publication |
|---|---|---|---|---|---|
| WO 9413350 | A | 23-06-1994 | AUCUN | | |
| EP 0778042 | A | 11-06-1997 | AT | 189965 T | 15-03-2000 |
| | | | AU | 692407 B2 | 04-06-1998 |
| | | | AU | 7413496 A | 26-06-1997 |
| | | | CA | 2191619 A1 | 05-06-1997 |
| | | | DE | 69606833 D1 | 06-04-2000 |
| | | | DE | 69606833 T2 | 15-06-2000 |
| | | | JP | 3533299 B2 | 31-05-2004 |
| | | | JP | 9276412 A | 28-10-1997 |
| | | | JP | 2001157713 A | 12-06-2001 |
| | | | JP | 2004136103 A | 13-05-2004 |
| | | | NO | 965156 A | 05-06-1997 |
| US 4991602 | A | 12-02-1991 | AUCUN | | |
| EP 0515201 | A | 25-11-1992 | CA | 2069052 A1 | 22-11-1992 |
| | | | DE | 69221937 D1 | 09-10-1997 |
| | | | DE | 69221937 T2 | 05-02-1998 |
| | | | ES | 2106139 T3 | 01-11-1997 |
| | | | HK | 1002626 A1 | 04-09-1998 |
| | | | JP | 3494666 B2 | 09-02-2004 |
| | | | JP | 6169996 A | 21-06-1994 |
| | | | JP | 2004041754 A | 12-02-2004 |
| | | | US | 5368049 A | 29-11-1994 |
| EP 0410557 | A | 30-01-1991 | AU | 5014790 A | 31-01-1991 |
| | | | CA | 2009798 A1 | 26-01-1991 |
| | | | JP | 3060674 A | 15-03-1991 |
| WO 2004093659 | A | 04-11-2004 | EP | 1615576 A2 | 18-01-2006 |
| | | | US | 2004249368 A1 | 09-12-2004 |
| | | | US | 2008065066 A1 | 13-03-2008 |
| EP 1803484 | A | 04-07-2007 | CA | 2571635 A1 | 27-06-2007 |
| | | | JP | 2007190376 A | 02-08-2007 |
| | | | US | 2007149951 A1 | 28-06-2007 |
| US 2003163064 | A1 | 28-08-2003 | AU | 2003216365 A1 | 09-09-2003 |
| | | | EP | 1487526 A1 | 22-12-2004 |
| | | | WO | 03072179 A1 | 04-09-2003 |
| | | | US | 2007021775 A1 | 25-01-2007 |
| US 2008051721 | A1 | 28-02-2008 | AUCUN | | |
| EP 1092449 | A | 18-04-2001 | CA | 2336416 A1 | 09-11-2000 |

EPO FORM P0460

Pour tout renseignement concernant cette annexe : voir Journal Officiel de l'Office européen des brevets, No.12/82

**EP 2 149 386 A1**

**ANNEXE AU RAPPORT DE RECHERCHE EUROPEENNE
RELATIF A LA DEMANDE DE BREVET EUROPEEN NO.**  EP 08 16 1434

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

13-11-2008

| Document brevet cité au rapport de recherche | Date de publication | Membre(s) de la famille de brevet(s) | Date de publication |
|---|---|---|---|
| EP 1092449 A | | WO 0066211 A1<br>US 6610046 B1 | 09-11-2000<br>26-08-2003 |
| US 2005273147 A1 | 08-12-2005 | WO 2005117754 A1 | 15-12-2005 |

EPO FORM P0460

Pour tout renseignement concernant cette annexe : voir Journal Officiel de l'Office européen des brevets, No.12/82

# EP 2 149 386 A1

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2006039217 A